# EUROPÄISCHE PATENTSCHRIFT

(11) **EP 1 514 525 B1**
(45) Veröffentlichungstag und Bekanntmachung des Hinweises auf die Patenterteilung: **19.03.2008**
(21) Anmeldenummer: 04018088.7
(22) Anmeldetag: 30.07.2004
(51) Int. Cl.: A61F 2/06, A61F 5/56, A61M 16/04

(54) **Prothese zum Einsetzen in den Hals eines Lebewesens**
Prosthesis for introducing into the throat of a living being
Prothèse pour l'introduction dans la gorge d'un être vivant

(30) Priorität: 12.09.2003 DE 20314282 U; 24.10.2003 DE 20316405 U
(43) Veröffentlichungstag der Anmeldung: 16.03.2005
(73) Patentinhaber: Mahlo, Hans-Wolfgang, Dr. med., 78462 Konstanz (DE)
(72) Erfinder: Mahlo, Hans-Wolfgang, Dr. med., 78462 Konstanz (DE)
(74) Vertreter: Hiebsch, Gerhard F.

(56) Entgegenhaltungen:
- WO-A-02/055123
- DE-A- 19 501 363
- US-A- 4 863 477
- US-A- 5 911 756
- US-A1- 2003 149 488

## Beschreibung

Die Erfindung betrifft eine Prothese zum Einsetzen in den Hals eines Lebewesens -- insbesondere in einen menschlichen Rachenraum -- nach dem Oberbegriff des Anspruches 1.

Seit langem sind sogenannte Stimmprothesen bekannt, von denen eine beispielhaft in der US-A-4 435 853 beschrieben wird als Verweilprothese mit geringem Strömungswiderstand für die prothetische Stimmrehabilitation etwa nach operativem Entfernen des Kehlkopfes eines Menschen wegen eines Kehlkopfkarzinoms. Die Stimmprothese wird in einen künstlich geschaffenen Speiseröhren-Luftröhrengang (ösophagotracheale Fistel) eingeschoben zur Unterstützung des Atmens, um Luft zur Mundhöhle zu führen. Hier dient jene Stimmprothese als Bewegungshilfe in der Art eines Einwegeventils. Die Stimmprothese umfasst ein i. w. zylindrisches Gehäuse aus Silikonwerkstoff mit Ein- und Auslassöffnungen; die seitliche Einlassöffnung mündet in Gebrauchsstellung in die Luftröhre, die -- mit einer schwenkbar als Einwegventil wirkenden Membrane ausgestattete -- Auslassöffnung in die Speiseröhre. Diese Auslassöffnung ist von einem Rohrende mit zur Längsachse geneigter Endkante umgeben, die an einem Radialkragen endet; die Stimmprothese ist in der Speiseröhre durch einen Radialkragen gehalten und greift die vordere Halspartie etwa radial und liegt andernends der Halsfläche des Menschen mittels von ihr abkragender Radialflügel von außen an, welch letztere dann durch Klebebänder festgelegt werden. Diese Stimmprothese wird mittels eines in sie axial einsetzbaren Stabes in die Luftröhrenfistel eingeschoben, der nach der Positionierung zu entfernen ist.

Dokument DE-A-19501363 beschreibt eine Rohrprothese mit den technischen Merkmalen des Oberbegriffs aus Anspruch 1.

In Kenntnis dieses Standes der Technik hat sich der Erfinder das Ziel gesetzt, eine in den Rachenraum einsetzbare Prothese zur Verhinderung eines Rachenkollapses bei Schlaganfall od. dgl. Ereignissen zu schaffen. Insbesondere sollen die Therapie des Schlafapnoe-Syndroms und des obstruktiven Schnarchens ermöglicht sowie die Verhinderung von Folgen der Schlafapnoe -- wie zerebraler Schlaganfall, Herzyrhythmusstörungen, Herzinfarkt und Bluthochdruck -- erreicht werden.

Zur Lösung dieser Aufgabe führt die Lehre des unabhängigen Anspruches; die Unteransprüche geben günstige Weiterbildungen an. Zudem fallen in den Rahmen der Erfindung alle Kombinationen aus zumindest zwei der in der Beschreibung, der Zeichnung und/oder den Ansprüchen offenbarten Merkmale. Bei angegebenen Bemessungsbereichen sollen auch innerhalb der genannten Grenzen liegende Werte als Grenzwerte offenbar und beliebig einsetzbar sein.

Erfindungsgemäß weist die Prothese ein Rohr mit netzartiger Wandung aus flexiblem Werkstoff auf, das an Knotenpunkten des Netzwerkes mit Saugnäpfen versehen ist. Bevorzugt ist das Netzwerk aus Silikonwerkstoff hergestellt mit an diesen angeformten Saugnäpfen.

Es hat sich gezeigt, dass dank der Luftdurchlässigkeit des Netzwerkes sowie die Saugfähigkeit der Saugnäpfe eine unerwünschte Verlagerung der Prothese -- und dadurch ein mögliches Ersticken des Prothesenträgers -- verhindert wird. Die aus weichem Material bestehende Prothese wird in den Rachenraum des Prothesenträgers eingesetzt und durch die Saugnäpfe gehalten.

Nach einem weiteren Merkmal der Erfindung ist das Rohr so gestaltet, dass von seinen Endkanten Endbereiche mit -- gegenüber einem Mittelabschnitt -- verändertem Querschnitt ausgehen.

Die wesentliche Ausgestaltung wird dadurch gekennzeichnet, dass beidends eines rohrförmigen Mittelabschnittes Rohrendabschnitte reduzierten Querschnittes angeformt sind. Es entsteht somit im axialen Rohrmittelabschnitt ein geschlossener Rohrraum, wohingegen die Endabschnitte rinnenförmig ausgestaltet sind. Dabei hat es sich als günstig erwiesen, dem Mittelabschnitt ein Viertel der Länge des Rohres zuzumessen. Diese Länge entspricht auch der des in Einbaustellung unteren Endbereiches der Prothese.

Bei einer anderen Ausgestaltung der Prothese soll zwischen zwei rohrförmigen Endbereichen ein Mittelabschnitt reduzierten Querschnittes angeordnet sein.

Als günstig hat es sich erwiesen, zur weiteren Befestigungsmöglichkeit die Prothese durch längenveränderlich Fixationsbänder od. dgl. Organe mit einer Zahnschiene zu verbinden, die bevorzugt teilkreisförmig gekrümmt ist und auf eine Zahnreihe des Prothesenträgers aufgesetzt zu werden vermag.

Es kann auch ein Zungenadapter vorgesehen werden aus einem Steg sowie einem Kunststoffring, der mit zumindest einem Faden ebenfalls im Zahnbereich des Prothesenträgers fixiert wird. Als Fixiermittel können zudem Hautklebestreifen eingesetzt werden.

Mit dieser erfindungsgemäßen Rachenhülse als Rachenschienung wird die Aufgabe in bestechender Weise gelöst, einen Rachenkollaps bei obstruktivem Schnarchen und dem Schlafapnoe-Syndrom hintanzuhalten.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in:
- Fig. 1, 6:: eine skizzenhafte Seitenansicht des Mund- und Halsbereiches eines Menschen mit angedeuteter Halsprothese;
- Fig. 2, 3:: jeweils eine Schrägsicht auf die Halsprothese;
- Fig. 4:: die Draufsicht auf die Halsprothese;
- Fig. 5:: eine Schrägsicht auf ein Zusatzelement.

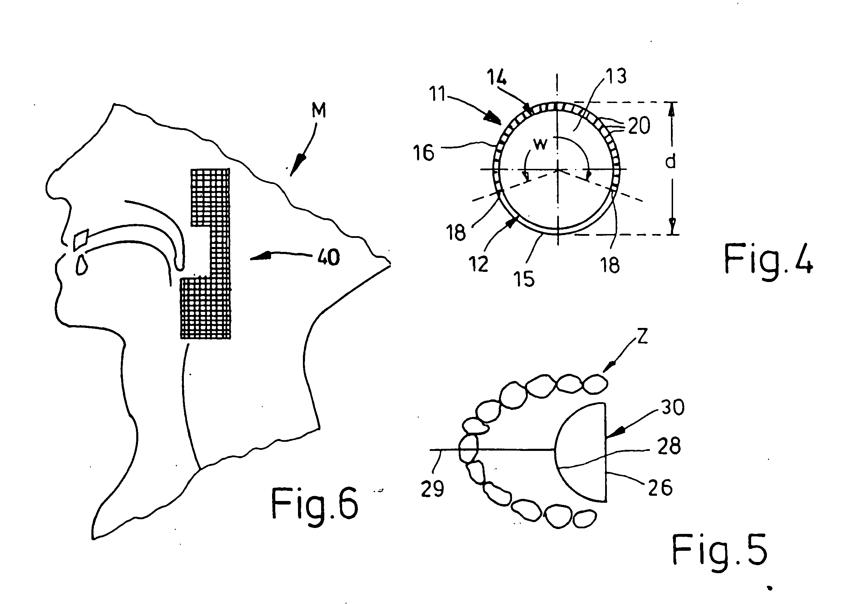

Eine Prothese 10 zur Einführung in den nicht weiter gezeigten Rachenraum eines Menschen M weist ein Rohr 11 axialer Länge a und eines Durchmessers d auf, von dem im Anschluss an jede seiner Endkanten 16 -- bis zu einem Mittelabschnitt 12 der Länge a₁ -- ein Teil seines Querschnitts entfernt worden ist. Diese Länge a₁ entspricht -- ebenso wie die Länge e des unteren Rohrendbereichs 14ₜ -- beispielsweise einem Viertel der Gesamtlänge a. Fig. 4 verdeutlicht, dass die Längskanten 18 der beiden Rohrendbereiche 14, 14ₜ zum Rohrraum 13 hin einen Mittelpunktswinkel w von hier 140° bestimmen. Diese Paare von Längskanten 18 enden jeweils an einer teilkreisförmigen Endkante 15 des Mittelstücks 12.

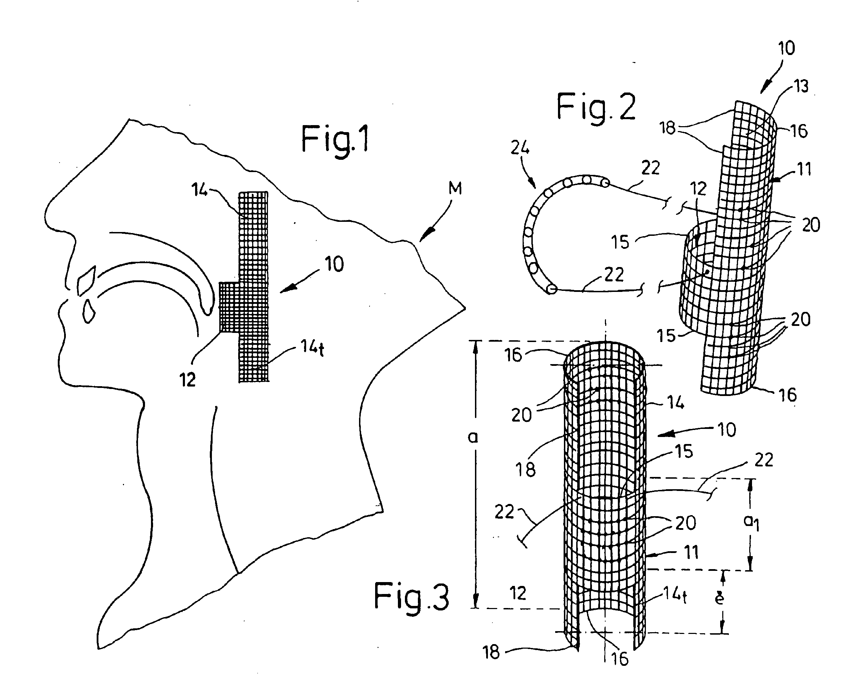

Das Rohr 11 ist aus einem luftdurchlässigen Silikonnetz hergestellt, in dessen Knotenpunkten Saugnoppen 20 angeformt sind, die eine Fixierung der Prothese 10 auf der Schleimhaut des Prothesenträgers M ermöglichen. Aus Gründen der Übersichtlichkeit sind in Fig. 2, 3 nur einige der Saugnoppen 20 kenntlich gemacht. Als weitere Befestigungsorgane sind an der oberen Endkante 15 des Mittelbereichs 12 einends längeneinstellbare Fixationsbänder 22 angebracht, die gemäß Fig. 2 mit einer Zahnschiene 24 verbunden sind. Letztere wird auf der unteren Zahnreihe des Prothesenträgers M festgelegt.

Die aus weichem Werkstoff geformte Prothese 10 wird in den Hals eingesetzt und drückt die Rachenwände zum Rücken hin, die Zunge wird lippenwärts gehalten. Das Netzwerk des Rohres 11 mit den Saugnoppen 20 verhindert ein unerwünschtes Lösen der Prothese 10.

Gemäß Fig. 5 wird ein Zungenadapter 30 eingesetzt aus einem Steg 26 und einem Kunststoffring 28, an dem ein flexibler Faden 29 festliegt. Letzterer wird an -- beispielsweise den Zähnen Nr. 31, 41 -- der bei Z angedeuteten Zahnreihe festgelegt. Statt des Steges 26 kann auch hier eine Silikonhülse vorgesehen werden.

Schließlich deutet die Skizze der Fig. 6 an, dass auch andere Ausgestaltungen der Rohrabschnitte 12, 14, 14ₜ bei einer Prothese 40 möglich sind.

## Patentansprüche

1. Prothese zum Einsetzen in den Hals eines Lebewesens, insbesondere in einen menschlichen Rachenraum, mit einem rohrartigen Bereich,
**dadurch gekennzeichnet,**
**dass** die Prothese (10, 40) ein Rohr (11) mit netzartiger Wandung aus flexiblem Werkstoff aufweist, das an Knotenpunkten des Netzwerkes mit Saugnäpfen (20) versehen ist.

2. Prothese nach Anspruch 1, **gekennzeichnet durch** ein Netzwerk aus Silikonwerkstoff sowie an diesen angeformte Saugnäpfe (20).

3. Prothese nach Anspruch 1 oder 2, **dadurch gekennzeichnet, dass** von den Endkanten (16) des Rohres (11) Endbereiche (14, 14ₜ) mit gegenüber einem Mittelabschnitt (12) verändertem Querschnitt ausgehen.

4. Prothese nach Anspruch 3, **gekennzeichnet durch** einen rohrförmigen Mittelabschnitt (12) der Länge (a₁), an den beidends jeweils ein Rohrendabschnitt (14, 14ₜ) reduzierten Querschnitts angeformt ist (Fig.2).

5. Prothese nach Anspruch 4, **dadurch gekennzeichnet, dass** die Längskanten (18) des Rohrendabschnittes (14, 14ₜ) in dessen Rohrinnenraum (13) einen radialen Mittelpunktswinkel (w) von mehr als 90° bestimmen.

6. Prothese nach Anspruch 4 oder 5, **gekennzeichnet durch** eine Länge (a₁) des Mittelabschnitts (12), die etwa einem Viertel der Rohrlänge (a) entspricht.

7. Prothese nach einem der Ansprüche 4 bis 6, **dadurch gekennzeichnet, dass** die Länge (e) des einen der Endbereiche (14ₜ) etwa einem Viertel der Rohrlänge (a) entspricht.

8. Prothese nach Anspruch 3, **dadurch gekennzeichnet, dass** zwischen zwei rohrförmigen Endbereichen (14, 14ₜ) der Prothese (40) ein Mittelabschnitt (12) reduzierten Querschnitts angeordnet ist (Fig. 6).

9. Prothese nach einem der Ansprüche 1 bis 8, **dadurch gekennzeichnet, dass** sie durch Fixationsbänder (22) mit einer Zahnschiene (24) verbunden ist, wobei gegebenenfalls eine teilkreisartige gekrümmte Zahnschiene (24) vorgesehen ist, welche Verbindungsmittel zum Anschluss an Zähne eines Prothesenträgers (M) enthält.

10. Prothese nach einem der voraufgehenden Ansprüche, **dadurch gekennzeichnet, dass** ihr ein Zungenadapter (30) aus einem Steg (26) sowie einem Kunststoffring (28) zugeordnet ist, der wenigstens einen Faden (29) zum Festlegen im Zahnbereich (Z) eines Prothesenträgers (M) aufweist, wobei gegebenenfalls, der Steg (26) von einer Silikonhülse gebildet wird mit verstärkten Enden zum Festlegen des Kunststoffringes (28).

## Claims

1. Prosthesis for insertion into the neck of a living being, in particular into a human pharynx, having a tubular region, **characterised in that** the prosthesis (10, 40) has a tube (11) with a net-like wall made from flexible material, which is provided with suction cups (20) at points of intersection of the network.

2. Prosthesis according to claim 1, **characterised by** a network made from silicone material and suction cups (20) moulded onto the latter.

3. Prosthesis according to claim 1 or 2, **characterised in that** end regions (14, 14ₜ) of modified cross-section with respect to a central section (12) start from the end edges (16) of the tube (11).

4. Prosthesis according to claim 3, **characterised by** a tubular central section (12) of length (a₁), to which at both ends in each case a tube end section (14, 14ₜ) of reduced cross-section is moulded (Figure 2).

5. Prosthesis according to claim 4, **characterised in that** the longitudinal edges (18) of the tube end section (14, 14ₜ) in its tube interior (13) determine a radial central point angle (w) of more than 90°.

6. Prosthesis according to claim 4 or 5, **characterised by** a length (a₁) of the central section (12), which corresponds to about one quarter of the tube length (a).

7. Prosthesis according to one of claims 4 to 6, **characterised in that** the length (e) of one of the end regions (14ₜ) corresponds to about one quarter of the tube length (a).

8. Prosthesis according to claim 3, **characterised in that** a central section (12) of reduced cross-section is arranged between two tubular end regions (14, 14ₜ) of the prosthesis (40) (Figure 6).

9. Prosthesis according to one of claims 1 to 8, **characterised in that** it is connected to an anchor splint (24) by fixing tapes (22), wherein optionally an anchor splint (24) curved like a graduated circle is provided, which contains connecting means for connection to teeth of a prosthesis support (M).

10. Prosthesis according to one of the preceding claims, **characterised in that** a tongue adapter (30) comprising a bar (26) and a plastic ring (28) is assigned to it and the tongue adapter (30) has at least one thread (29) for fixing in the tooth region (Z) of a prosthesis support (M), wherein optionally the bar (26) is formed by a silicone sleeve with reinforced ends for fixing the plastic ring (28).

## Revendications

1. Prothèse destinée à être introduite dans le cou d'un être vivant, en particulier dans un espace de pharynx humain, avec une zone tubulaire,
**caractérisée en ce que**
la prothèse (10, 40) présente un tube (11) avec une paroi réticulaire à base de matériau flexible, qui est doté de ventouses (20) sur des points nodaux du réseau.

2. Prothèse selon la revendication 1, **caractérisée par** un réseau à base de matériau silicone ainsi que des ventouses (20) formées sur le matériau.

3. Prothèse selon la revendication 1 ou 2, **caractérisée en ce que** des zones d'extrémité (14, 14ₜ) avec une section modifiée par rapport à une partie centrale (12) partent des arêtes d'extrémité (16) du tube (11).

4. Prothèse selon la revendication 3, **caractérisée par** une partie centrale (12) tubulaire de longueur (a₁), sur laquelle une partie d'extrémité de tube (14, 14ₜ) de section réduite est formée respectivement sur les deux extrémités (figure 2).

5. Prothèse selon la revendication 4, **caractérisée en ce que** les arêtes longitudinales (18) de la partie d'extrémité du tube (14, 14ₜ) déterminent dans son espace intérieur de tube (13) un angle au sommet (w) radial supérieur à 90°.

6. Prothèse selon la revendication 4 ou 5, **caractérisée par** une longueur (a₁) de la partie centrale (12), qui correspond à peu près à un quart de la longueur de tube (a).

7. Prothèse selon l'une quelconque des revendications 4 à 6, **caractérisée en ce que** la longueur (e) de l'une des zones d'extrémité (14ₜ) correspond à peu près à un quart de la longueur de tube (a).

8. Prothèse selon la revendication 3, **caractérisée en ce qu'**une partie centrale (12) de section réduite est disposée entre deux zones d'extrémité (14, 14ₜ) tubulaires de la prothèse (40) (figure 6).

9. Prothèse selon l'une quelconque des revendications 1 à 8, **caractérisée en ce qu'**elle est reliée par des bandes de fixation (22) à un segment denté (24), un segment denté (24) incurvé en forme de cercle partiel étant prévu éventuellement, lequel segment denté contient des moyens de liaison pour le raccordement à des dents d'un support de prothèse (M).

10. Prothèse selon l'une quelconque des revendications précédentes, **caractérisée en ce qu'**un adaptateur de langue (30) à base d'une nervure (26) et d'une bague plastique (28) lui est attribué, laquelle bague présente au moins un fil (29) pour la fixation dans la zone de dent (Z) d'un support de prothèse (M), la nervure (26) étant formée éventuellement par un manchon en silicone avec des extrémités renforcées pour la localisation de la bague plastique (28).
